# EUROPEAN PATENT SPECIFICATION

(11) **EP 2 722 075 B1**
(45) Date of publication and mention of the grant of the patent: **28.02.2018**
(21) Application number: 12189453.9
(22) Date of filing: 22.10.2012
(51) Int. Cl.: A61Q 19/00, A61Q 19/02, A61Q 19/08, A61K 8/97

(54) **Prevention of conditions arising from an impaired skin barrier function**
Vorbeugung von trockener Haut und Linderung für empfindliche Haut
Prévention de conditions de peau sèche et apaisement pour peaux sensibles

(43) Date of publication of application: 23.04.2014
(73) Proprietor: BASF Beauty Care Solutions France S.A.S., 69366 Lyon Cedex 07 (FR)
(72) Inventor: Laloeuf, Aurelie, L-2420 Luxembourg (LU)
(74) Representative: Mendelsohn, Isabelle M. N.

(56) References cited:
- WO-A2-2012/083906
- FR-A1- 2 864 446
- GR-A- 20050 100 386
- KUMARASAMY Y ET AL: "Screening seeds of Scottish plants for antibacterial activity", MEDICINAL & AROMATIC PLANTS ABSTRACTS, SCIENTIFIC PUBLISHERS, SCIENTIFIC PUBLISHERS, NEW DELHI - INDIA, vol. 25, no. 3, 31 December 2003 (2003-12-31), XP018016694, ISSN: 0250-4367

## Description

### Field of the Invention

The invention relates to cosmetic compositions for skin care by external application. More specifically, the invention relates to plant derived active agents for such compositions which enhance the skin barrier function while combating ageing effects resulting from skin dehydration.

### Background to the Invention

The skin is a vital organ which covers the entire surface of the body and ensures protective, sensitive, immune, metabolic or thermoregulatory functions. Water is essential to the normal functioning of the skin and must be carefully regulated. The skin barrier function is important as it plays a key role in protecting the body against water loss and external aggressions, the side effect of which include ageing. One of the effects of the process of ageing on the skin is dehydration and increased risk of irritation or sensitization of the skin.

Skin has three layers (i) the epidermis, (ii) the dermis, and (iii) the subcutaneous tissues. The epidermis forms the top layer of human skin and has the roles including acting as a mechanical barrier to protect the internal organs of the body, generating new skin cells, protecting against UV radiation, and fending off antigens.

The epidermis is the upper layer of the skin and is composed of and functions as a vital barrier between internal and external environments (Eckert RL 1989). The epidermis contains highly specialized epithelial cells predominated by keratinocytes, but melanocytes and Langerhans cells are also present. A number of structural proteins such a filaggrin, keratin and protease enzymes, lipids and antimicrobial peptides present contribute to maintaince of healthy barrier function, hydration levels and microbe resistance of the skin.

The arrangement of the specialized cells in the skin layers is delimited by an intercellular lipid structure comprising free cholesterol, cholesterol ionic (sulfate cholesterol), ceramide and other free fatty acids. The water impermeability is due to the conformation of the lipid molecules and its components within the skin layers.

The top layer of the epidermis is known as the stratum corneum, which forms the "bricks and mortar" of the skin. The stratum corneum functions to control regulation of the moisture content of the skin. The "brick" portion of the stratum corneum is keratin protein, which provides structure and strength to the skin. The "mortar" between the keratin protein is comprised of skin lipids (phospholipid bilayer), which produce the fluid structure of the cell membranes of the living layers of the epidermis. The lipid structure is organized in specific lamellar liquid crystal phases which form the intracellular cement of the stratum corneum and are essential for the water exchanges and healthy barrier function of the epidermis.

Keratinocyte cells synthesize the major structural components of the epidermal barrier through a process of programmed differentiation known as keratinization. Keratinocyte differentiation involves the process of cell cycle arrest known as cornification and the onset of expression of numerous genes resulting in the formation of four layers of epideramal cells: stratum basale, stratum spinosum, stratum granulosum and stratum corneum (Blumenberg M 1997). The synthesis of important structural and catalytic proteins critical to health of the skin barrier occurs during keratinocyte differentiation, these include: keratin, fillagrin, transglutaminase and involucrin. In fact, involucrin is a protein marker for keratinocyte differentiation appearing in the upper layers of the epidermis. Cornification is the process of forming an physical epidermal barrier in stratified squamous epithelial tissue. Keratinization is part of this process, in which the keratinocytes generate keratin until they undergo programmed cell death to form corneocytes. Corneocytes are completely keratinized dead cells composed of keratinocytes in the terminal stage of their differentiation. As the keratinocytes in the basal layer of the epidermis undergo the process of continuous and oriented maturation, the corneocytes are formed. The fully cornified keratinocytes then form the outermost layer of the skin barrier, which are constantly shed off and replaced by new cells over an average renewal / turnover time for the epidermis is 21 days.

At the cellular level, cornification is characterised by terminal keratinocytes differentiation, the production of keratin and keratinocytes metabolism ceasesation. During keratinocyte differentiation, phospholipids in the skin are gradually replaced by a mixture composed predominantly of lipids or fatty acids, of cholesterol and of ceramides (sphingolipids). The lamellar arrangement of the lipids of the lipid domain of the epidermis and the corneocytes participate in the epidermal barrier function. In healthy skin, the keratin and lipids join together to provide the skin barrier that keeps irritants out and the appropriate amount of moisture in. Together, with the arrangement of the corneocytes, the intercellular lipid domains of the stratum corneum provide the skin its high water impermeability characteristics.

Furthermore, keratinocytes assist in forming the barrier against environmental damage such as pathogens, heat, UV radiation and water loss. If pathogens start to invade the upper layers of the epidermis, keratinocytes can react with the invaders to lead to the production of proinflammatory mediators (such as chemokines) which attract leukocytes to the site of pathogen invasion. Keratinocytes are also potent immune moduoators and producers of anti-inflammatory mediators such as IL-10 and TGF-β. When activated, they can stimulate cutaneous inflammation and Langerhans cell activation via TNFα and IL-1β secretion. Keratinocytes also offer protection against damage from ultraviolet radiation (UVR) by taking up melanosomes, vesicles containing the endogenous photoprotectant melanin, from epidermal melanocytes. Melanin is then stored in the keratinocytes' nuclei, where it protects the DNA from UV damage.

An alteration or negative disruption of the skin barrier function increases trans-epidermal water loss and may trigger different skin conditions such a dryness, itchiness or dermatitis, inflammation and injury, all of which can contribute to poor skin appearance and ageing. Furthermore, it is clear that the quality of the cutaneous barrier depends on complex endogenous biological mechanisms involving many proteins, growth factors, adhesion molecules, hormones, lipids and lipid metabolism enzymes. A detrimental change in the cutaneous barrier can occur in the presence of other external attacks, such as irritants, mechanical, heat or climatic imbalances or xenobiotics or of internal attacks, such as psychological stress to result in dry and/or irritated skin.

Of the skin three compartments, dermis is the most deeply altered during the process of ageing, but the other components are not spared modification. Epidermal thickness is reduced, whilst, at the same time, the thickness of the horny layer increases. Keratinocyte renewal is slower, but their terminal differentiation is also slowed down. Epidermis hydration is decreased and skin barrier function decreases. Langerhans cells are less active, so immunity is reduced, meaning greater sensitivity to irritations, inflammation and infections. Combating skin dehydration of the skin associated with ageing requires counteracting the loss of skin barrier function. Therefore, ageing skin must be kept well moisturized.

Dry skin occurs when the skin loses its lipids, or the "mortar" in the stratum corneum. When lipids are missing, external irritants and potentially pathogenic organisms can travel between the keratin into lower layers of the epidermis. Missing lipids also allow moisture in the skin to travel to the surface where it evaporates. The result is dry, irritated skin that can be uncomfortable and unsightly. The biggest contributing factor to dry skin is weather and central heating. Symptoms of dry, irritated and/or inflamed skin may include redness and irritation, flaking, itching, cracking, and fissuring.

There are several dry skin management strategies that have explored the use of emollient formulations mixed with plant, algae and/or vitamin active ingredients to improve skin barrier condition (Vicanova J 2006, Grether-Beck S 2008, Eichenfield LF 2009).

Moisture can be held in the skin by applying a product to its surface that blocks water from leaving. Examples of ingredients that may help create this barrier on the surface of the skin include petrolatum, high concentrations of glycerin or other silicones, dimethicone, and mineral oil. Adding a barrier also helps to provide an environment where dry skin more rapidly repairs itself, and may provide some protection to the skin from irritants or other harmful materials. Some moisturizing products contain synthetic biomemetic lipids which penetrate the stratum corneum and fill spaces where skin lipids are missing. These naturally derived synthetic lipids mimic skin lipids by attracting water into the skin and by helping to maintain the proper balance of oil and water in the skin.

Many types of cosmetic composition have been used to treat dry skin. Typically, cosmetic compositions contain suitable active agents that compensate for dehydration function by increasing the amout of water present in the stratum coreum. Typically, these compositions contain lipophilic moisturizing agents that inhibit water loss via occlusion. Suitable agents include polyols such as glycerol or glycols, or by use of agents that protect the hydrolipid film of the skin by preventing evaporation of water by a barrier effect, such agents include hydrocarbon substances such as liquid petroluem. However, the moisturization afforded by polyols is limited in time and barrier forming agents take time to build up. Humectants can also be used to increase moisture levels in the skin. Humectants are ingredients such as glycerin that attract, retain, and hold moisture from the air. Unfortunately, such moisturisation techniques may only lead to temporary relief from dry skin. More favourable are techniques that repair and/or increase the skin barrier function, as these can result in the skin feeling more moisturised for longer and can benefit the health of the skin by repair. Healthy skin barrier can retain moisture and higher levels of hydration.

Prior art compositions comprising lipophilic moisturizing agents, humectants and active ingredients are known, however, they do not perform sufficiently well to benefit those with very dry skin. Because the barrier function of the dry skin is compromised, any moisture that is added to the skin may quickly be lost again. Therefore, moisturizers without barrier properties to help prevent moisture loss may need to be applied more frequently.

*Armeria maritima* is a species of perennial flowering plant otherwise known as thrift, sea thrift, or sea pink. The plant can be found in the wild in coastal areas across the Northern Hemisphere, especially in Europe, in area of dry, sandy, saline conditions such as those at beaches, costal marches and cliffs. Sea thrift is rarely used in herbal medicine and is more generally thought of as a non-medicinal plant, although the dried flowering plant has antibiotic properties. The edible leaves can be used in the treatment of obesity, some nervous disorders and urinary infections. Extracts of the aerial parts of *Armeria maritima* are recognised as skin conditioners by the EU Commission ingredients database, see - http://ec.europa.eu/consumers/cosmetics/cosing/index.cfm?fuseaction=search.details v2&id=5450 3. *Armeria Maritima* has been used for its antioxidant properties in combination with other active ingredients in an antipollution role in a limited number of cosmetic products to protect the skin from heavy metals, pesticides and cigarette smoke.

Scientifically, skin moisturisation results from stimulation of epidermal differentiation and maturation of the *stratum corneum.* The keratinocyte proliferation in the basal layer, the innermost skin layer, differentiate gradually as they pass through the spinous layer and the granular layer. Through this keratinization process, the keratinocytes produce natural moisturizing factors (NMFs) and lipids (ceramides, cholesterols, and fatty acids), and form the stratum corneum, thereby contributing to a healthy skin barrier function and consequently hydrated skin, less prone to irritation and effects of aging. Thus, keratinocyte differentiation can therefore deliver skin moisturisation and resistance to irritation or inflammation and ageing by providing the epidermis with the necessary cells and constituents to promote healthy skin barrier function and associated efficient water management within the skin. Therefore, means for inducing keratinocyte differentiation in epidermis would be highly desirable to counteract dehydration, dry skin conditions, effects of ageing and irritated and inflamed or otherwise sensitive skin.

One means of improving skin moisturisation involves controlling the semi-permeability or moisture retaining capability of the skin is by enhancing epidermal barrier function and renewal. The semi-permeability of the skin, which in turn plays a key role in skin moisturisation and protection. However, external damages and/or intrinsic factors can affect this semi-permeability leading to dry and/or sensitive skin conditions arising. Therefore in addition to delivering moisturisation, one must also control the inflammatory and irritation processes that can occur in order to efficiently promote healthy skin barrier function, which gives rise to healthy skin.

It is clear that the skin's internal or endogenous mechanism of maintaining moisturisation is centered around barrier function optimal functioning. The skin barrier function plays a key role in protecting the body against skin water loss and external aggressions. An alteration of the skin barrier function increases trans-epidermal water loss and leads to dry and flaky skin, inflammation and injuries.

A good moisturiser should therefore (i) enhance keratinocyte differentiation to provide the skin with the necessary cells and constituents to increase the water content throughout the different layer of the skin of the epidermis and the *stratum corneum, (ii)* increase water transport, water channel proteins and natural moisturising factors, which participate in promoting efficient water management and (iii) maintain an optimal skin lipid balance by increasing the synthesis of new lipids. The present invention proposes to accomplish same. Patent document FR 2864446 discloses the use of a lyophilisate of de-differentiated plant cells in the regeneration, lightening and depigmentation of the skin.

### Summary of the Invention

According to the present invention, as set out in the appended claims, there is provided an *Armeria maritima* plant extract for use in the treatment and/or prevention of dry skin conditions caused by impaired skin barrier function.

By impaired skin barrier function, it is meant alteration or negative disruptions of the skin barrier function which lead to weakening of the skin barrier and associated loss of moisturizing lipids and increases in trans-epidermal water loss. Impaired skin barrier function can manifest itself by triggering skin conditions such as dryness, flakiness, itchiness and dermatitis, inflammation, redness or sensitization by pathogen invasion. All of these factors can contribute to poor skin appearance and premature ageing.

The invention relates to the finding that *Armeria maritima* plant derived active agents present in extracts of the flowers; leaves; stems; roots or other parts of the plant are effective at enhancing the health of the skin barrier function.

By enhancing the enhancing the health of the skin barrier function, it is meant promotion of skin keratinocyte differentiation in the skin to promote healthy skin barrier function through an increase in keratinization processes in the skin. Promoted keratinization leads to better strateum corneum production through cornification and concurrent production of natural moisturizing factors (NMFs) and lipid production (ceramides, cholesterols, and fatty acids) associated with keratinization in the skin. Increased kertain and lipid production promote impermeability of the skin barrier and therefore assist the skin in resisting moisturizing lipid loss, dehydration and invasion by pathogens.

Promotion of keratinocyte differentiation thus contributes to a healthy skin barrier function and consequently hydrated skin, which is less prone to microbe and irritant invasion through improved barrier function. Furthermore, promotion of keratinocyte differentiation can counter act the natural slowing of the keratinization process, which occurs on ageing, providing means for the skin to appear more youthful.

Thus, keratinocyte differentiation can therefore delivers skin moisturisation and resistance to irritation or inflammation and ageing by providing the epidermis with the necessary cells and constituents to ensure healthy skin barrier function and associated efficient lipid and water management within the skin. Improving or promoting healthy skin barrier function therefore provides relief and/or prevention of skin conditions arising from excess moisture loss from skin. Such skin conditions include dryness, flakiness, itchiness and dermatitis, inflammation, redness or sensitization. Symptoms of dry, irritated and/or inflamed skin may include redness and irritation, flaking, itching, cracking, and fissuring.

The *Armeria maritima* plant extract for use as a promoting agent for keratinocyte differentiation in skin tissue or cell culture.

Advantageously, the *Armeria maritima* plant extract for use as a maturation agent for the *stratum corneum* in skin tissue or cell culture. A person skilled in the art will appreciate that acting as a maturation agent provides benefit by way of improved barrier function.

Additionally, the extract has been found to combating skin stress resulting from inflammation and irritation by decreasing levels of cortisol found in the skin.

The *Armeria maritima* plant extract for use as an inhibiting agent and/or reducing agent for cortisol production in skin tissue or cell culture.

The effects arising from the extract on skin barrier function through promoted keratinocyte differentiation, stratum corneum maturation and decreasing levels of cortisol, increase and/or promote healthy skin barrier function and reduce skin irritation and inflammation.

The improvement in skin barrier function and skin stress relief leading to a lessening of ageing effects resulting from skin dehydration and inflammation.

In a preferred embodiment the extract of the invention is an aqueous extract. Desirably, the extract is prepared from the flowers; leaves; stems; roots or any other part of *the Armeria maritima* plant not otherwise mentioned.

The extract of the invention may be used advantageously as a cosmetic agent for skin care by external application to the skin or indeed in *in-vitro* tests involving cell cultures.

Thus, a preferred aspect of the invention relates to cosmetic application of the extract of the invention for skin care by external application.

Thus in a related aspect, there is provided for use of an *Armeria Maritima* plant extract as an agent in the preparation of a composition for increasing skin barrier function. In a preferred embodiment, the composition is a cosmetic composition.

In a preferred embodiment, there is provided for such a use wherein increasing skin barrier function prevents and/or treats a dry skin condition and/or sensitive skin condition caused by skin irritation and inflammation.

Suitably, such cosmetic use or medical use of the extract of the invention includes external application to the skin to reduce skin damage, prevent or improve skin aging, prevent or improve wrinkles, improve skin elasticity, improve skin firmness, to whiten skin, to moisturise the skin, to prevent or improve inflammation or soothe/relieves irritation, to improve or rejuvenate skin complexion and texture, and/or to improve skin radiance and/or luminescence, promote luminescence and/or improves skin radiance.

In another preferred aspect of the invention, a cosmetic composition for external application to the skin comprising an effective amount of *Armeria maritima* plant extract as an active agent for the treatment and/or prevention of dry skin conditions and/or skin irritation and inflammation (involving impaired skin barrier function) is provided.

In a preferred embodiment, the extract of the invention comprises from about 1 - 10mg of plant material per 1ml of solution, most preferably, the extract contains 5mg of plant material per 1 ml of aqueous solution (%w/v).

Suitably, the cosmetic composition of the invention comprises an amount of the *Armeria maritima* plant extract in the composition between 0.01 to 1 wt %, and preferably 0.05 to 0.5 wt % based on the total weight of the composition.

The composition compositions comprising the extract can advantageously be used to reduces skin damage, prevent or improve skin aging, prevent or improve wrinkles, improves skin elasticity, improves firmness, is used for skin whitening, skin moisturizing, prevents or improves inflammation or relieves irritation, improve skin complexion, improves radiance/luminescence, promotes luminescence and/or improves skin radiance.

The composition of the invention may be provided as a thickened solution, a gel, an ointment, a lotion, a cream, a milk, a foam, a spray, an emulsion, a dispersion, a powder, a paste or a solid stick.

The composition of the invention may further comprise cosmetic adjuvants selected from the group consisting of: fatty substances, organic solvents, silicones, thickeners, emollients, UVA, UVB or broad band sunscreens, antifoam agents, hydrating agents, perfumes, stabilizers, surfactants, fillers, sequestrants, anionic, cationic, non ionic or amphoteric polymers, propellants, alkalifying or acidifying agents, dyes and metal oxide pigments, or mixtures thereof.

In a preferred embodiment, the composition of the invention may further comprise agents for protecting the skin from free radical damage. Thus, according to a preferred embodiment of the invention, the cosmetic composition may be a sun protection composition, i.e. a composition assisting in the protection against solar radiation. Thus, actives assisting in solar protection, such as for example, solar filters, can be advantageously added to the composition according to the invention.

The Armeria maritima plant extracts and compositions comprising effective amounts of same, according to the invention will be able to be applied by any cosmetically suitable manner, in particular compositions for external topical application. Their formulation will be readily adapted by the man skilled in the art depending on the cosmetic use desired.

Advantageously, the cosmetic compositions according to the invention are intended for an administration in a topical cutaneous manner. The preferred compositions therefore contain a cosmetically acceptable medium, i.e. compatible with the skin and the appendages. In particular, the preferred compositions will be able to be in the form of creams, oil-in-water emulsions, or water-in-oil or multiple emulsions, solutions, suspensions, gels, milks, lotions, foams, sticks or else powders, suited to an application on the skin, scalp, the lips and/or the appendages. They can be used as a care product and/or as a make-up product for the skin.

These compositions comprise the necessary excipients for their formulation, such as solvents, thickeners, thinners, surfactants, antioxidants, colouring agents, preservatives, perfumes. The necessary excipients will be known to the skilled person and include solvents, thickeners, thinners, antioxidants, colouring agents, solar filters, suntan simulating agents, pigments, charges, preservatives, perfumes, odour absorbers, cosmetic or pharmaceutical actives, essential oils, vitamins, essential fatty acids, surfactants, film-forming polymers, etc.

Advantageously, a preferred composition of the invention may contain other active principles intended to promote its action. Among these other active principles, the active principles can be named which have an anti-radical or antioxidant action, for example, vitamin C, vitamin E, the coenzyme Q10 and polyphenolic plant extracts, etc.

In particular, the active principle according to the invention will be able to be used advantageously in a cosmetic preparation intended to combat in a preventive and/or curative manner against the manifestations of cutaneous ageing involving poor or dysfunctional skin barrier function.

The active principle according to the invention, or the composition containing it, will allow one to combat, in particular, against the loss of elasticity and of firmness of the skin.

The active principle according to the invention allows the skin and the appendages to be protected against all types of external aggressions. The use of the active principle, or of a composition containing it, will allow the skin and the appendages to be protected and to better resist environmental stresses.

The invention further consists of a method of cosmetic treatment intended to give a healthier appearance to the skin and to improve the brightness and radiance of the complexion, characterized by the application on the skin which is to be treated of a composition containing an effective quantity of active extract of the invention to skin promote or restore healthy skin barrier function.

However, in case of skin diseases or troubles, the normal function of the *stratum corneum* is not maintained because of several reasons which can lead to cutaneous discomfort which can be characterized by tightness, smarting, inflammation and/or itching.

As a result, the skin barrier is damaged, resulting in skin dryness and, in severe cases, inflammations. In such skin disease-related inflammations the extract and compositions comprising same reduced cortisol levels in the skin to reduce irritation and inflammation.

This disclosure is directed to providing a screening method involving the Armeria maritima extract or composition comprising same, capable of effectively screening a material for improving skin functions which improves skin barrier function, promotes skin moisturization, or prevents skin aging.

In particular, the active extract of the invention can be used advantageously in a cosmetic preparation intended to combat in a preventive and/or curative manner against the manifestations of cutaneous ageing arising from dehydration and/or problems with skin barrier function. Cutaneous manifestations of ageing are understood to mean all modifications of the exterior appearance of the skin and the appendages due to ageing, such as, for example, wrinkles and fine lines, withered skin, flabby skin, thinned skin, the lack of elasticity and/or of tonus of the skin, dull skin without brightness, or pigmentation blemishes of the skin.

The active extract of the invention, or the composition containing it, will allow one to combat, in particular, against the loss of elasticity and of firmness of the skin.

The active principle according to the invention allows the skin and the appendages to be protected against all types of external aggressions. The use of the active principle, or of a composition containing it, will allow the skin and the appendages to be protected and to better resist environmental stresses which cause irritated or inflamed or sensitized skin, particularly in delicate skin associated with poor barrier function.

The expression "external aggression" is understood to mean the aggressions which can be produced by the environment. By way of example, one can name aggressions such as pollution, UV rays, or else products of an irritant character such as surfactants, preservatives or perfumes. Pollution is understood to mean both "external" pollution, due for example to diesel particles, ozone or heavy metals, and "interior" pollution which can be due in particular to the emissions of solvents of paints, glues or wallpapers (such as toluene, styrene, xylene or benzaldehyde), or else cigarette smoke.

In a related aspect there is provided a cosmetic skin care method for enhancing the barrier function of the skin, and/or enhancing moisturization of the skin, and/or strengthening the resistance of the skin to internal and/or external stress, and/or enhancing the brightness of the skin, comprising a step of applying the cosmetic composition of the invention to the skin to achieve the beneficial effects recited herein.

The invention also encompases use of an *Armeria Maritima* plant extract as a cosmetic agent for stimulating kerinatocyte differentiation and/or inhibiting cortisol production in the skin in the preparation of a composition for the prevention and/or treatment of a dry skin and/or sensitive skin condition caused by skin irritation and inflammation.

### Definitions

By the term "skin conditioning" it is meant that the skin is maintained in good condition; this incorporates but is not limited to skin moisturisation/hydration, anti-oxidant, anti-pollution and anti-aging modes of action,

By the term "skin moisturization", it is meant that the water content of the skin is increased, rather than just maintained at existing levels. This could be through attracting moisture into the skin layers and/ or by creating a barrier to retain moisture within the skin.

### Brief Description of the Drawings

Figure 1 illustrates: Ameria Martima induced involucrin expression within keratinocytes
**Figure 2** **illustrates Involucrin ng/ug Total protein**

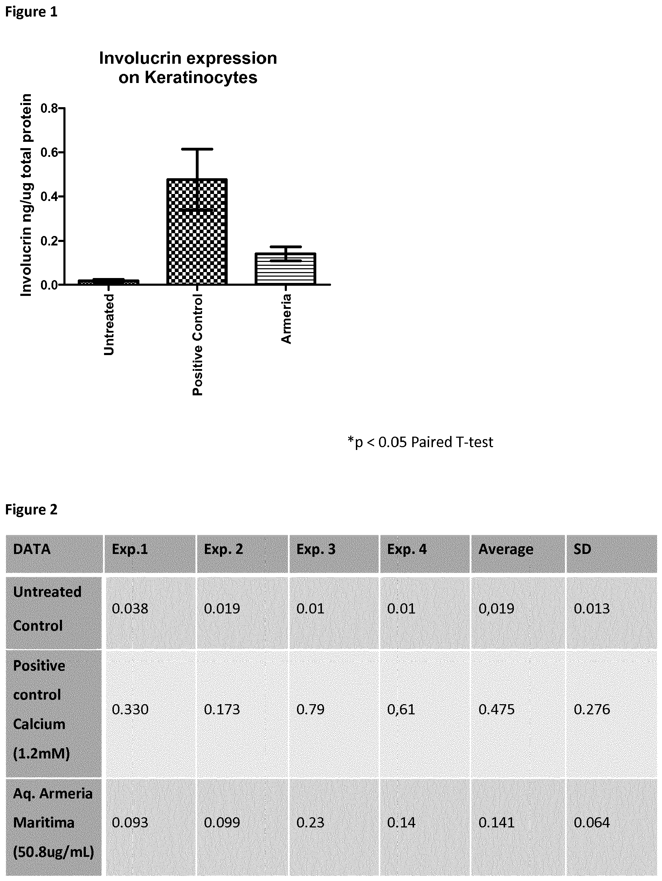

Figure 2 illustrates: Armeria Martima induced cortisol attenuation

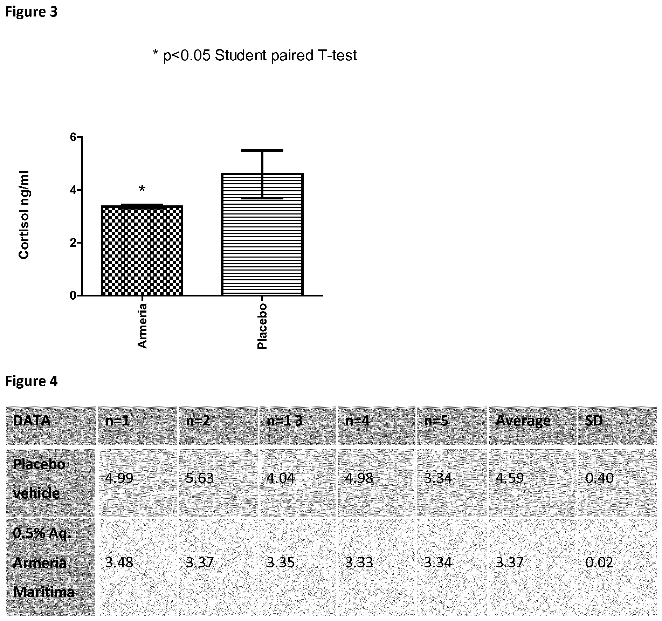

**Figure 4****: Cortisol ng/ml**

### Detailed Description of the Invention

Advantages and characteristics of the invention will be better apparent from reading examples, given by way of illustration and not restrictively.

### • Example 1

### Preparation of Active Principle (Extract) from Armeria maritima

The active principle is obtained from plants of the species *Armeria maritima.* Armeria Maritima was extracted using a supercritical CO² method and was formulated at 0.5% in a 1,2-pentanediol (Hydrolite® 5, Symrise).

### • Example 2

### Study: Exploration of the ability of an aqueous extract of Armeria maritima to influence key epidermal differentiation markers

### Obiective

The objective of this study was to investigate whether topical application of aqueous *Armeria maritima* extract influences the following:
(i) Keratinocyte differentiation to provide the skin with the necessary cells and biochemical constituents to increase the water content throughout the different layers of the skin of the epidermis and the stratum corneum,
(ii) Water transport, water channel proteins and natural moisturising factors, which participate in promoting efficient water management, and
(iii) Synthesis of new lipids in the skin.

### Procedure

*In vitro* keratinocyte monolayer cell cultures were treated with *Armeria maritima* aqueous extract (0.5%wt) to determine ability *of Armeria Maritima* to induce Keratinocyte diffrentiation.

Keratinocyte primary cells (Invitrogen) were cultured *in vitro* in KGM-2 media (Lonza) containing calcium at a low passage (p2). Four days prior to intended treatment with active ingredients cells were switched to a low calcium media (0.03mM). This low calcium media was used for culturing for the remainder of the experiment.

Cells were treated with either Aq *Armeria maritima* reconstituted in KGM-2 media, calcium chloride 1.2mum as a positive control or KGM-2 media only. Cell media containing treatments was removed and replaced with fresh low calcium media containing active ingredient/positive control/media only after 3 days. On the 6^{th} day supernatants from each cell treatment were removed and adhered cells were harvested using a combination of Tissue Protein Extraction Reagent (T-PER, Piercenet) and manual scrapping. The harvested cell extracts were then boiled in eppendorfs to 100°C for 10mins. Involucrin is a protein marker for keratinocyte differentiation appearing in the upper layers of the epidermis. Involucrin expression was measured after 6 days in *Armeria Maritima* treated cells and untreated cells (n=4) using Bioplex (SkinMap Involucrin kit) following the manufacturer's instructions.

### Results

Involucrin expression is induced after 3 days treatment (n=2; data not presented).

Increased involucrin expression indicates that keratinocyte differentiation occurred in cultures upon treatment with the aqueous *Armeria maritima* extract. Involucrin expression was increased by 419% compared to control 'untreated' cells. This significant increase in involucrin expression indicates a substantial increase in keratinocyte differentiation within *in vitro* keratinocyte monolayer cultures.

### Conclusion

This data indicates that *Armeria maritima* aqueous plant extract is active in the stimulation of keratinocyte differentiation, and produces associated changes in the keratinocyte differentiation protein biomarker profile. This indicates the capacity of *Armeria maritima* to induce cellular differentiation in the skin.

### • Example 3

### Study: Gene array studies on Armeria maritima extract (links to moisturisation benefits)

### Procedure

### Culture of reconstructed epidermis (RHE) and topical application of Armeria Maritima aqueous extract:

An aqueous extract of Armeria Maritima was formulated at 0.5% in a 1,2-pentanediol (Hydrolite® 5, Symrise) and added topically on reconstructed epidermis (SkinEthic, Nice, France) at a volume of 5 ul/0.5 cm² for 24 hours. Culture medium was supplied by SkinEthic and used according to supplier's recommendation. Oleanolic acid at the same concentration was used as a positive control. The study was placebo controlled using 1,2-pentaneglycol at the same volume.

### RNA Preparation

Total RNA was isolated from human skin samples using a commercial kit (RNeasy Mini Kit, Qiagen, Chatsworth CA) according to the manufacturer's protocol. The skin tissues were mechanically disrupted and homogenized using the tissue lyser (Qiagen). For detailed protocol please see protocol *SP-SR-017.*

### Microarray expression analysis

RNA concentration was measured with ND-1000 spectrophotometer (NanoDrop Technologies, Wilmington, DE) and RNA quality was evaluated using the Agilent 2100 Bioanalyzer system (Agilent Technologies Inc, Palo Alto, CA).
200 ng of total RNA from each sample were used to generate amplified and biotinylated sense-strand cDNA from the entire expressed genome according to the Ambion WT Expression Kit(P/N 4425209 Rev B 05/2009) and Affymetrix GeneChip® WT Terminal Labeling and Hybridization User Manual (P/N 702808 Rev. 1, Affymetrix Inc., Santa Clara, CA). GeneChip® ST Arrays (GeneChip® Human Gene 1.0 ST Array) were hybridized for 16 hours in a 45°C incubator, rotated at 60 rpm. According to the GeneChip® Expression Wash, Stain and Scan Manual (PN 702731 Rev 2, Affymetrix Inc., Santa Clara, CA) the arrays were then washed and stained using the Fluidics Station 450 and finally scanned using the GeneChip® Scanner 3000 7G.

### Microarray data analysis

Analysis of the gene expression data was carried out in the statistical computing language R (http://www.r-project.org) using packages available from the Bioconductor project (www.bioconductor.org). The raw data was normalized using the robust multi-array average (RMA) method first suggested by Li and Wong in 2001 (1, 2). In order to search for the differentially expressed genes in RHE's stimulated with the *Armeria maritima* extract compared to placebo an empirical Bayes moderated t-test was then applied (3), using the 'limma' package (4). To address the problem with multiple testing, the p-values were adjusted using the method of Benjamini and Hochberg (Benjamini Y, 1995).

### Results

Gene array data showed an increased expression (> 1,4-fold) of 7 genes involved in keratinocyte differentiation following topical application of *Ameria Maritima.* These genes were analyzed manually. The upregulated genes were:
i. late cornified envelope 4A,
ii. cornulin,
iii. chromosome 1 open reading frame 46,
iv. hornerin /// repetin,
v. late cornified envelope 5,
vi. peptidyl arginine deiminase, type I and
vii. keratin 9.

The gene, aquaporin 5, was also upregulated which is involved in water management processes in the skin.

### Conclusion

The upregulation of genes involved in the keratinocyte differentiation process indicates the ability of *Armeria Maritima* to deliver skin moisturisation benefits associated with increased skin barrier function. This role in skin moisturisation is further supported by the effect of *Armeria Maritima* on Aquaporin 5.

The involucrin expression work combined with the gene array data indicates that *Armeria Maritima* aqueous extract has keratinocyte differentiation properties which are linked to improved skin moisturisation and improved skin barrier function and other anti-aging benefits linked to skin integrity.

### • Example 4

### Study: Gene array studies indicating further anti-aging benefits

### Procedure

Experimental method as described above.

### Results

Gene array data on topical application of *Armeria Maritima* on reconstructed epidermis showed upregulation of 8 genes within the biology ontology group of cellular respiration/ATP synthesis. This was shown by bioinformatics studies using DAVID bioinformatics software.

Genes found to be upregulated and forming part of the enriched cluster of cellular respiration/ATP synthesis coupled electron transport were:
1. Cytochrome b
2. Cytochrome c oxidase subunit 3; ATP synthase subunit; ATP synthase protein 8
3. NADH dehydrogenase (ubiquinone) 1, subcomplex unknown, 2, 14.5 kDa [NDUFC2]
4. NADH-ubiquinone oxidoreductase chain 4L
5. Catalase [CAT]
6. ATPase, Na+/K+ transporting, beta 3 polypeptide [ATP1B3]
7. Aldolase C, fructose-biphosphate [ALDOC]
8. Succinate-CoA ligase, alpha subunit [SUCLG1]

Upregulation of these associated genes can translate into the following cosmetic benefits:
I. Prevention of wrinkles due to increased cellular respiration and cellular metabolism and
II. Reduction of wrinkles,
III. Increased firmness,
IV. More revitalized skin,
V. Increased radiance/luminescence due to increased skin regeneration.

### Conclusion

Increased expression of genes involved in delivering the cosmetic benefits described above suggests a wider role for *America Maritima* in general skin condition.

### • Example 5

### Study: Cortisol expression studies (anti-stress, anti-inflammation benefits to the skin)

### Procedure

Protein quantification of cell marker, cortisol, was measured by Bioplex (SkinMap kit) following manufacturer's instructions. RHE tissues were treated for 24hrs with the formulation containing aqueous *Armeria maritima* extract. RHE tissue models are a 3D reconstructed human epidermis model consisting of normal primary human epidermal keratinocytes. Their structure resembles that of a natural epidermis with a basement membrane, proliferating keratinocytes and a stratum corneum that has barrier function. As a result of having this barrier, RHE models can be treated topically with a formulation.

### Results

Tested tissues showed a significant decrease in Cortisol production compared to placebo treated RHE tissues.

### Conclusion

Dry skin can often be accompanied by skin irritation and inflammation due to the skin barrier being compromised. Increased cortisol levels are found in irritated and inflamed skin as a result of stress factors affecting the skin.

The finding that aqueous extracts of *Armeria maritima* can reduce cortisol production in RHE tissue suggests additional anti-stress, anti-inflammatory properties of this aqueous plant extract. Reduction of elevated cortisol levels reduced inflammation, redness and other symptoms of irritated sensitized skin. This anti-inflammatory action provides anti-redness, skin lightening and anti-blemish benefits. Further enhancing barrier optimal functioning and the moisturising benefits of the composition.

The data presented here shows that the aqueous extracts of *Armeria Maritima* stimulates keratinocytes resulting in changes within their protein biomarker profile.

In vitro involucrin expression was significantly demonstrating the capacity of *Armeria Maritima* to induce cellular differentiation.

The inflammatory marker cortisol was found to be reduced in Armeria-treated RHE tissues indicating an anti-inflammatory effect of *Armeria Maritima* on these tissues.

In our study, the early differentiation marker Keratin 1, 10 (n=5) showed increased expression following *Armeria Maritima* treatment versus the placebo formulation.

Of note, no changes in involucrin expression was observed in RHE models after 24hrs of *Armeria Maritima* treatment (data not shown), this is due to the short treatment length of these tissues with *Armeria Maritima* as compared to the 6 day-treatment of the keratinocyte monolayers with *Armeria Maritima.*

Due to the nature of the RHE models it is not viably feasible to keep them in culture for 6 days upon arrival, hence, lengthy time course experiments are conducted on monolayer keratinocyte cells.

Moreover, the RHE models were treated for a 24hr period to keep in line with the treatment period of the RHE models treated in the gene array study.

Overall, the data shows that aqueous *Armeria Maritima* extract has the ability to improve and/or promote healthy skin barrier function (and vis-a-vis dry skin conditions) by way of stimulating keratinocyte differentiation, as shown in our *in vitro* experiments. Dry skin can often be accompanied by skin inflammation due to the skin barrier being compromised (Addor FA 2010). The finding that *Armeria Maritima* can reduce cortisol production suggests additional anti-inflammatory properties of this aqueous plant extract. The results support the anti-stress/anti-inflammation benefits proposed by this invention.

### Preparation of Compositions comprising an effective amount of Armeria Maritima

Example compositions comprising the *Armeria maritima* extract are provided, by way of illustration and are not intended to limit the invention in any way.

### • Example 6

The composition below describes oil in water cream formulation incorporating the *Armeria maritima* extract. The percentages indicated are by weight of the composition unless stated otherwise.

| | Wt% |
|---|---|
| Hydrogenated Polyisobutene | 4.5 |
| Cyclopentasiloxane | 4.5 |
| Dimethicone | 2.5 |
| Glyceryl stearate | 2 |
| Cetyl alcohol | 2 |
| PEG-40 Stearate | 1.25 |
| Sodium acrylate/acryloydimethyl taurate copolymer | 1 |
| Tocopheryl acetate | 0.5 |
| Preservatives | QS |
| Perfume | QS |
| Armeria maritima extract | 0.5 |
| Water | to 100 |

### • Example 7

The composition below describes a foundation formulation incorporating the *Armeria maritima* extract. The percentages indicated are by weight of the composition unless stated otherwise.

| | Wt% |
|---|---|
| Cyclopentasiloxane | 14 |
| Pigments | 9 |
| PolyGlyceryl-4-isotearate | 5 |
| Isodecane | 4 |
| Butylene glycol | 2 |
| Bis-PEG/PPG-14/14 dimethicone | 2 |
| Hydrogenated polyisobutene | 1.5 |
| Beeswax | 1.2 |
| Magnesium Sulphate | 1 |
| Perservatives | QS |
| Perfume | QS |
| Armeria maritima extract | 0.5 |
| Water | to 100 |

### • Example 8

The composition below describes an aqueous gel formulation incorporating the *Armeria maritima* extract. The percentages indicated are by weight of the composition unless stated otherwise.

| | Wt% |
|---|---|
| Carbomer | 0.8 |
| Glycerine | 6.0 |
| Glyceryl acrylate/acrylic acid copolymer | 0.4 |
| Sodium Hydroxide | 0.25 |
| Preservative | QS |
| Perfume | QS |
| Armeria Maritima extract | 0.5 |
| Water | To 100 |

### • Example 9

The composition below describes a body scent formulation incorporating the *Armeria maritima* extract. The percentages indicated are by weight of the composition unless stated otherwise.

| | Wt% |
|---|---|
| Denatured Alcohol | 55 |
| Propylene Glycol | 5 |
| Glycerine | 3 |
| Benzophenone-2 | 0.1 |
| Perfume | 6 |
| Solubiliser | 2 |
| Armeria maritima extract | 0.5 |
| Water | To 100 |

### References

Addor FA, Aoki V. Skin Barrier in atopic dermatitis. An Bras Dermatol 2010 85(2): 184-94. Blumenberg M, Tomić-Canić M. Human epidermal keratinocyte: keratinization processes. EXS 1997;78:1-29.
Eckert RL, Rorke EA. Molecular biology of keratinocyte differentiation. Environ Health Perspect. 1989 Mar;80:109-16.
Eichenfield LF, McCollum A, Msika P. The benefits of sunflower oleodistillate (SOD) in pediatric dermatology. Pediatr Dermatol. 2009, 26(6): 669-75.
Elias PM. Stratum corneum defensive functions: an integrated view. J Invest Dermatol 2005;125:183-200.
Grether-Beck S, Muhlberg K, Brenden H, Felsner I, Brynjolfsdottir A, Einarsson S, Krutmann J. Bioactive molecules from the Blue Lagoon: in vitro and in vivo assessment of silica mud and microalgae extracts for their effects on skin barrier function and prevention of skin ageing. Exp Dermatol 2008 17(9):771-9.
Toulza E, Mattiuzzo NR, Galliano MF, Jonca N, Dossat C, Jacob D, et al. Large-scale identification of human genes implicated in epidermal barrier function. Genome Biol 2007;8: R107.
Vicanova J, Bouex C, Lacroix S, Lindmark L, Damour O. 2006. Epidermal and dermal characteristics in skin equivalent after systemic and topical application of skin care ingredients. Ann N Y Acad Sci 2006, 1067:337-42.

## Claims

1. Non-therapeutic cosmetic use of Armeria Maritima plant extract as a cosmetic agent for skin care by external application to the skin to moisturize the skin.

2. Non-therapeutic use of an effective amount of Armeria maritima plant extract as an active agent for the treatment and/or the prevention of dry skin conditions in a cosmetic composition by external application to the skin.

3. Use according to the preceding claim in which the dry skin conditions involve impaired skin barrier function.

4. Use according to any one of the preceding claims in which the extract is an aqueous extract.

5. Use according to any one of the preceding claims wherein the extract comprises from 1-10mg of plant material per 1mls of aqueous solution (%w/v).

6. Use according to one of the claims 2 to 5 wherein the amount of Armeria maritima plant extract in the cosmetic composition is between 0.01 to 1.0 wt %;

7. Use according one of the claims 2 to 5 wherein the amount of Armeria maritima plant extract in the cosmetic composition is between 0.05 to 0.5 wt% based on the total weight of the composition.

8. Use according to one of the claims 2 to 7 wherein the cosmetic composition is provided as a thickened solution, a gel, an ointment, a lotion, a cream, a milk, a foam, a spray, an emulsion, a dispersion, a powder, a paste or a solid stick.

9. Use according to one of the claims 2 to 8 wherein the cosmetic composition contains a cosmetically acceptable medium.

10. A non-therapeutic cosmetic skin care method for enhancing moisturization of the skin comprising a step of applying the cosmetic composition according to any one of claims 2 to 9 to the skin.

## Patentansprüche

1. Nicht-therapeutische kosmetische Verwendung eines *Armeria maritima* Pflanzenextraktes als ein kosmetisches Mittel zur Hautpflege mittels äußerlicher Anwendung auf der Haut, um die Haut zu befeuchten.

2. Nicht-therapeutische Verwendung einer wirksamen Menge eines *Armeria maritima* Pflanzenextraktes als ein aktiver Inhaltsstoff zur Behandlung und/oder Vorbeugung trockener Hautzustände in einer kosmetischen Zusammensetzung mittels äußerlicher Anwendung auf der Haut.

3. Verwendung nach dem vorhergehenden Anspruch, wobei die trockenen Hautzustände eine gestörte Barrierefunktion der Haut umfassen.

4. Verwendung nach einem der vorhergehenden Ansprüche, wobei der Extrakt ein wässriger Extrakt ist.

5. Verwendung nach einem der vorhergehenden Ansprüche, wobei der Extrakt 1 bis 10 mg des Pflanzenmaterials pro 1 ml der wässrigen Lösung (% w/v) umfasst.

6. Verwendung nach einem der Ansprüche 2 bis 5, wobei die Menge des *Armeria maritima* Pflanzenextraktes in der kosmetischen Zusammensetzung 0,01 bis 1,0 Gew.-% beträgt.

7. Verwendung nach einem der Ansprüche 2 bis 5, wobei die Menge des *Armeria maritima* Pflanzenextraktes in der kosmetischen Zusammensetzung basierend auf dem Gesamtgewicht der Zusammensetzung 0,05 bis 0,5 Gew.-% beträgt.

8. Verwendung nach einem der Ansprüche 2 bis 7, wobei die kosmetische Zusammensetzung als eine verdickte Lösung, ein Gel, eine Salbe, eine Lotion, eine Creme, eine Milch, ein Schaum, ein Spray, eine Emulsion, eine Dispersion, ein Pulver, eine Paste oder ein fester Stift bereitgestellt wird.

9. Verwendung nach einem der Ansprüche 2 bis 8, wobei die kosmetische Zusammensetzung ein kosmetisch akzeptables Medium enthält.

10. Nicht-therapeutisches kosmetisches Hauptpflegeverfahren zum Verbessern der Befeuchtung der Haut, umfassend einen Schritt des Anwendens der kosmetischen Zusammensetzung nach einem der Ansprüche 2 bis 9 auf der Haut.

## Revendications

1. Utilisation cosmétique non thérapeutique d'un extrait de plante *Armeria maritima* en tant qu'agent cosmétique pour le soin de la peau par application externe sur la peau pour hydrater la peau.

2. Utilisation non thérapeutique d'une quantité efficace d'un extrait de plante *Armeria maritima* en tant qu'agent actif pour le traitement et/ou la prévention des conditions de la peau sèche dans une composition cosmétique par application externe sur la peau.

3. Utilisation selon la revendication précédente, dans laquelle les conditions de la peau sèche incluent une fonction barrière de la peau altérée.

4. Utilisation selon l'une quelconque des revendications précédentes, dans laquelle l'extrait est un extrait aqueux.

5. Utilisation selon l'une quelconque des revendications précédentes, dans laquelle l'extrait comprend de 1 à 10 mg de matière végétale pour 1 ml de solution aqueuse (% p/v).

6. Utilisation selon l'une quelconque des revendications 2 à 5, dans laquelle la teneur en extrait de plante *Armeria maritima* dans la composition cosmétique est entre 0,01 et 1,0 % en poids.

7. Utilisation selon l'une quelconque des revendications 2 à 5, dans laquelle la teneur en extrait de plante *Armeria maritima* dans la composition cosmétique est entre 0,05 et 0,5 % en poids par rapport au poids total de la composition.

8. Utilisation selon l'une quelconque des revendications 2 à 7, dans laquelle la composition cosmétique est fournie sous forme d'une solution épaisse, d'un gel, d'une pommade, d'une lotion, d'une crème, d'un lait, d'une mousse, d'un spray, d'une émulsion, d'une dispersion, d'une poudre, d'une pâte ou d'un bâtonnet solide.

9. Utilisation selon l'une quelconque des revendications 2 à 8, dans laquelle la composition cosmétique contient un milieu cosmétiquement acceptable.

10. Procédé cosmétique non thérapeutique de soin de la peau pour augmenter l'hydratation de la peau comprenant une étape d'application sur la peau de la composition cosmétique selon l'une quelconque des revendications 2 à 9.
